Europäisches Patentamt

(19) European Patent Office

Office européen des brevets

(11) Publication number : **0 357 186 B1**

(12) **EUROPEAN PATENT SPECIFICATION**

(45) Date of publication of patent specification :
**28.10.92 Bulletin 92/44**

(21) Application number : **89306321.4**

(22) Date of filing : **22.06.89**

(51) Int. Cl.⁵ : **A61K 7/48,** A61K 7/06

(54) **Skin and hair-care compositions.**

(30) Priority : **25.06.88 GB 8815180**

(43) Date of publication of application :
**07.03.90 Bulletin 90/10**

(45) Publication of the grant of the patent :
**28.10.92 Bulletin 92/44**

(84) Designated Contracting States :
**AT BE CH DE ES FR GB GR IT LI LU NL SE**

(56) References cited :
**EP-A- 0 006 232
AU-B- 2 847 177
FR-A- 2 192 798
FR-A- 2 192 799
GB-A- 1 461 991
US-A- 3 903 258
US-A- 4 675 177**

(73) Proprietor : **BEECHAM GROUP PLC
Beecham House Great West Road
Brentford Middlesex TW8 9BD (GB)**

(72) Inventor : **Fairhurst, Edgar Beecham Products
Research Department St. George's Avenue
Weybridge Surrey KT13 0DE (GB)**
Inventor : **Sayers, Helen Beecham Products
Research Department St. George's Avenue
Weybridge Surrey KT13 0DE (GB)**
Inventor : **Poile, Steven Beecham Products
Research Department St. George's Avenue
Weybridge Surrey KT13 0DE (GB)**

(74) Representative : **West, Vivien et al
Beecham Pharmaceuticals Great Burgh Yew
Tree Bottom Road
Epsom Surrey KT18 5XQ (GB)**

EP 0 357 186 B1

EP 0 357 186 B1

## Description

This invention relates to improvements in or relating to skin- and hair-care compositions containing silica.

The incorporation of silica into skin- and hair-care compositions for other purposes is known. For example, EP-A-0 118 180 discloses the use of silanised silica gel as an anti-seborrheic agent, and EP-A-0 117 621 discloses its use in a deodorant.

FR-A-2192799, FR-A-2192 798 and US-A-3903258 disclose antiperspirant compositions in which aluminium salts are dispersed in liquid esters including, inter alia, triethylcitrate and triethyl acetyl citrate. The examples show the use of small amounts (0.35% by wt) of silica as a suspending agent. GB-A-1461991 describes similar compositions in which the carrier is an ester of formula $R-OOC-R_1-COOR$, where R is an alkyl group having 2 to 6 carbon atoms and $R_1$ is a hydroxy substituted saturated alkylene radical having 4 to 8 carbon atoms and 2 6o e hydroxyl groups. The examples disclose use of 0.2% by weight silica as a suspending agent.

A disadvantage of previously proposed compositions that are not loaded with antiperspirant salts is that the level of silica has been limited by its tendency to become visible as an unsightly residue on hair or areas of dry (non-greasy) skin.

It has now surprisingly been found that this problem can be overcome by the incorporation into the composition of esters of citric, tartaric and succinic acid.

Such esters are commercially available, and some, the $\alpha$-hydroxy derivatives, have been proposed for use in the treatment of skin disorders such as dandruff. They have also been used as carriers for antiperspirant salts, as described above. They are non-volatile and have the texture of a light oil. However, they are soluble to varying degrees in aqueous alcohols, and most show significant water solubility.

Furthermore, they have been found to have the same refractive index as commercially available silicas. As a result, when the silica particles are coated with one of these esters, during which process any air pockets are automatically excluded, they become transparent. The present invention is based on these findings.

A first aspect of the invention therefore provides a skin- or hair-care composition comprising at least 0.5% w/w silica, a $C_{2-4}$ alkyl ester of citric, tartaric or succinic acid, wherein any hydroxy residue is optionally acetylated, and an aqueous carrier therefor.

Preferred alkyl esters include ethyl and butyl.

Advantageously, the ester is selected from triethyl citrate, acetyl triethyl citrate, tributyl citrate, acetyl tributyl citrate, diethyl tartrate and diethyl succinate. Preferably the ester is triethyl citrate or tributyl citrate or their acetylated derivatives.

A particular advantage of the esters of the invention is that, unlike other commonly available oils, they have a significant degree of solubility in aqueous solvent systems. Also, unlike many carboxylic acid esters, such as ethyl lactate, they are resistant to hydrolysis when suspended in water or aqueous alcohols. Thus they can be formulated into a stable non-oily composition.

Thus, a further aspect of the invention provides a skin- or hair-care composition comprising at least 0.5% w/w silica, and an ester as defined above dissolved in an aqueous solvent system.

Typically the aqueous solvent system is an aqueous alkanol or water, preferably aqueous ethanol.

Preferably ethanol is included in the composition at from 2% to 80% w/w, more preferably 10% to 70% w/w, especially about 30% w/w in antisebborheic and anti-acne preparations, and about 70% w/w in deodorant preparations.

On application to the skin or hair, the aqueous solvent system evaporates. Once out of solution, the ester becomes associated with the silica particles and masks them by rendering them transparent as described above. The association is particularly strong in the case of hydrophobic silicas such as silanised silica gel, making it particularly suitable for incorporation into compositions of the type described in EP-A-0 118 180.

Surprisingly, it has been found that the anti-seborrheic properties of silanised silica gel remain largely unaffected by adsorption of the esters of the invention in the amounts required to mask the presence of the silica.

As indicated above, the silica in the compositions of the invention is preferably a hydrophobic silica such as silanised silica gel. However, it is envisaged that the improvement of the present invention may be applied to any skin- or hair-care composition containing silica, for example as a microencapsulating agent for other active ingredients, or as a deodorant ingredient in which particulate silica is included to increase the evaporative surface area and rapidly remove sweat.

The discovery on which the present invention is based has been found to apply to a wide range of commercially available hydrophilic and hydrophobic silicas, including silica gels and precipitated silicas such as pyrogenic (fumed) silica; examples include Cab-O-Sil M-5, Sipernat D10, Sipernat D17 and Aerosil R972 (all Trade Marks).

Suitably the silica has a mean particle size of 0.1 to 20μm, the particles consisting of aggregates of smaller

2

particles.

Preferably the composition comprises at least 0.5% w/w of the silica, more preferably about 2%. The proportion of silica will not normally exceed 10% w/w, more preferably about 5% w/w.

The amount of ester will normally be selected to give an ester:silica ratio of at least 1:1, preferably about 2:1. Thus, the composition will typically contain at least 1% w/w, preferably about 4% w/w, ester. In the above-mentioned aqueous compositions of the invention, the amount of ester will not normally exceed the solubility limit thereof, which will obviously vary according to the ester used, the solvent system used, and the other ingredients present, and may be as high as 30% w/w. However, it is normally preferred that the ester be present in an amount of up to 20% w/w, more preferably up to 10% w/w.

As used herein, 'skin- or hair-care composition' denotes any composition which is to be applied to the skin and/or hair for its beneficial therapeutic and/or cosmetic properties, including organoleptic properties.

Suitably the composition is presented in a conventional skin- or hair-care form, for instance as a roll-on formulation, gel, cream, lotion or stick, and will contain conventional carriers and diluents known to be suitable for each particular presentation.

The compositions may also comprise optional accessory ingredients such as perfume, colouring and preserving agents, keratolytics, antibacterials and antimicrobial agents such as Irgasan or chlorhexidine including its salts such as chlorhexidine gluconate.

Preferably the composition is presented as an aqueous ethanolic gel.

A process for the preparation of a composition of the invention comprises admixing the ingredients thereof in the required proportions.

A method for the therapeutic and/or cosmetic treatment of skin and/or hair comprises applying a composition according to the invention to the skin and/or hair.

The antiseborrheic compositions of the invention are typically applied to the skin, usually the face, on a regular (1-3 times per day) basis. They can be formulated as a leave-on product or as a wipe-on, wipe-off cleansing product which is optionally impregnated into absorbent material for use as swabs.

The following Examples illustrate the invention:

Example 1

Oil-free Gel (for seborrhea)

| Ingredient | | % w/w |
|---|---|---|
| Silanised silica gel | : | 2.0 |
| Triethyl citrate | : | 4.0 |
| Ethanol | : | 30.0 |
| *Carbopol 940 | : | 0.5 |
| Triethanolamine | : | 1.2 |
| *Nipastat | : | 0.3 |
| Butylparahydroxybenzoate: | | 0.02 |
| *Germall 115 | : | 0.3 |
| Deionised water | to | 100 |

Example 2

Antimicrobial Gel (for mild acne)

| Ingredient | | % w/w |
|---|---|---|
| Silanised silica gel | : | 1.0 |
| Triethyl citrate | : | 2.0 |
| Ethanol | : | 30.0 |
| Chlorhexidine gluconate | : | 0.5 |
| *Carbopol 940 | : | 0.5 |
| Triethanolamine | : | 1.2 |
| *Nipastat | : | 0.3 |
| Butylparahydroxybenzoate | : | 0.02 |
| *Germall 115 | : | 0.3 |
| Deionised water | to | 100 |

*Trade Marks.

Example 3

Roll-on Lotion (deodorant)

| Ingredient | | % w/w |
|---|---|---|
| *Cab-O-Sil M-5 | : | 1.0 |
| Tributyl Citrate | : | 3.0 |
| Ethanol | : | 70.0 |
| Irgasan (Triclosan) | : | 0.5 |
| *Methocel E4M | : | 2.0 |
| *Nipastat | : | 0.3 |
| Butylparahydroxybenzoate | : | 0.02 |
| *Germall 115 | : | 0.3 |
| Deionised water | to | 100 |

Example 4

Matting Emulsion

| Ingredient | | % w/w |
|---|---|---|
| *Arlacel 60 | : | 1.5 |
| *Tween 60 | : | 2.0 |
| *Arlacel 165 | : | 5.0 |
| Tributyl citrate | : | 8.0 |
| *Cab-O-Sil M-5 | : | 3.0 |
| *Dimethicone 350 | : | 2.0 |
| *Nipastat | : | 0.3 |
| Butylparahydroxybenzoate: | | 0.02 |
| *Germall 115 | : | 0.3 |
| Deionised water | to | 100 |

*Trade Marks.

**Claims**

1. A skin-care or hair-care composition comprising at least 0.5% w/w silica, a $C_{2-4}$ alkyl ester of citric, tartaric or succinic acid, optionally acetylated at any hydroxy residue, and an aqueous carrier.

2. A composition according to claim 1, in which the aqueous carrier is an aqueous solvent system.

3. A composition according to claim 2, in which the aqueous solvent system is an aqueous lower alkanol or water.

4. A composition according to claim 3, in which the aqueous solvent system is aqueous ethanol.

5. A composition according to any preceding claim, comprising up to 10% w/w silica.

6. A composition according to claim 4, comprising 2 to 5% w/w silica.

7. A composition according to any preceding claim, in which the ester is selected from triethyl citrate, acetyl triethyl citrate, tributyl citrate, acetyl tributyl citrate, diethyl tartrate and diethyl succinate.

8. A composition according to any preceding claim, in which the silica has a mean particle size of 0.1 to 20μm, the particles consisting of aggregates of smaller particles.

9. A composition according to any preceding claim, in which the ester: silica ratio is at least 1:1.

10. A composition according to any preceding claim, in which the silica is a hydrophobic silica.

11. A composition according to claim 10, in which the silica is silanised silica gel.

12. The use of a $C_{2-4}$ alkyl ester of citric acid, tartaric acid or succinic acid, optionally acetylated at any hydroxy residue, in a skin-care or hair-care composition comprising at least 0.5% w/w silica in an aqueous carrier, to mask the presence of silica on evaporation of the carrier.

## Patentansprüche

1.  Hautpflege- oder Haarpflegemittel, umfassend mindestens 0,5 Gew.-% Silika, einen $C_{2-4}$-Alkylester der Citronen-, Wein- oder Bernsteinsäure, gegebenenfalls acetyliert an irgendeinem Hydroxylrest, und einen wäßrigen Träger.

2.  Mittel nach Anspruch 1, bei dem der wäßrige Träger ein wäßriges Lösungsmittelsystem ist.

3.  Mittel nach Anspruch 2, bei dem das wäßrige Lösungssystem ein wäßriges niedriges Alkanol oder Wasser ist.

4.  Mittel nach Anspruch 3, bei dem das wäßrige Lösungssystem wäßriges Ethanol ist.

5.  Mittel nach einem der vorstehenden Ansprüche, umfassend bis zu 10 Gew.-% Silika.

6.  Mittel nach Anspruch 4, umfassend 2 bis 5 Gew.-% Silika.

7.  Mittel nach einem der vorstehenden Ansprüche, wobei der Ester ausgewählt ist aus Triethylcitrat, Acetyltriethylcitrat, Tributylcitrat, Acetyltributylcitrat, Diethyltartrat und Diethylsuccinat.

8.  Mittel nach einem der vorstehenden Ansprüche, wobei das Silika eine mittlere Teilchengröße von 0,1 bis 20 μm aufweist, wobei die Teilchen aus Aggregaten kleinerer Teilchen bestehen.

9.  Mittel nach einem der vorstehenden Ansprüche, in dem das Verhältnis des Esters zum Silika mindestens 1:1 beträgt.

10. Mittel nach einem der vorstehenden Ansprüche, wobei das Silika ein hydrophobes Silika ist.

11. Mittel nach Anspruch 10, in dem das Silika ein silanisiertes Silikagel ist.

12. Verwendung eines $C_{2-4}$-Alkylester von Citronen-, Wein- oder Bernsteinsäure, gegebenenfalls acetyliert an einem Hydroxylrest, in einem Hautpflege- oder Haarpflegemittel, umfassend mindestens 0,5 Gew.-% Silika in einem wäßrigen Träger, um die Gegenwart des Silikas beim Verdampfen des Trägers zu maskieren.

## Revendications

1.- Composition pour le soin de la peau ou des cheveux comprenant au moins 0,5 % en p/p de silice, un ester alkylique en $C_{2-4}$ d'acide citrique, tartrique ou succinique, dont un quelconque résidu hydroxy est éventuellement acétylé, et un support aqueux.

2.- Composition suivant la revendication 1, dans laquelle le support aqueux est un système solvant aqueux.

3.- Composition suivant la revendication 2, dans laquelle le système solvant aqueux est un alcanol inférieur aqueux ou de l'eau.

4.- Composition suivant la revendication 3, dans laquelle le système solvant aqueux est de l'éthanol aqueux.

5.- Composition suivant l'une quelconque des revendications précédentes, comprenant jusqu'à 10 % en p/p de silice.

6.- Composition suivant la revendication 4, comprenant 2 à 5 % en p/p de silice.

7.- Composition suivant l'une quelconque des revendications précédentes, dans laquelle l'ester est choisi parmi le citrate de triéthyle, le citrate d'acétyltriéthyle, le citrate de tributyle, le citrate d'acétylbutyle, le tartrate de diéthyle et le succinate de diéthyle.

8.- Composition suivant l'une quelconque des revendications précédentes, dans laquelle la silice a une dimension particulaire moyenne de 0,1 à 20 μm, les particules consistant en agrégats de particules plus petites.

9.- Composition suivant l'une quelconque des revendications précédentes, dans laquelle le rapport ester/silice est d'au moins 1/1.

10.- Composition suivant l'une quelconque des revendications précédentes, dans laquelle la silice est une silice hydrophobe.

11.- Composition suivant la revendication 10, dans laquelle la silice est un gel de silice silané.

12.- Utilisation d'un ester alkylique en $C_{2-4}$ d'acide citrique, d'acide tartrique ou d'acide succinique, dont un quelconque résidu hydroxy est éventuellement acétylé, dans une composition pour le soin de la peau ou des cheveux comprenant au moins 0,5 % en p/p de silice dans un support aqueux, pour masquer la présence de la silice après évaporation du support.